# EUROPEAN PATENT SPECIFICATION

(11) **EP 0 496 409 B1**
(45) Date of publication and mention of the grant of the patent: **16.10.1996**
(21) Application number: 92101132.6
(22) Date of filing: 24.01.1992
(51) Int. Cl.: C12Q 1/04

(54) **A method for the detection of gram-negative bacteria**
Ein Verfahren zum Nachweis gramnegativer Bakterien
Une méthode de détection de bactéries gram-négatifs

(30) Priority: 24.01.1991 FI 910359
(43) Date of publication of application: 29.07.1992
(73) Proprietor: ORION CORPORATION ORION DIAGNOSTICA, SF-02200 Espoo (FI)
(72) Inventor: Tuompo, Helena, 02150 Espoo (FI); Glasin, Heljä, 02780 Espoo (FI)
(74) Representative: VOSSIUS & PARTNER

(56) References cited:
- EP-A- 0 107 594
- JOURNAL OF CLINICAL MICROBIOLOGY, vol. 26, no. 7, 01 July 1988, Washington, DC (US); S. ROMERO et al., pp. 1378-1382/
- METHODS OF ENZYMATIC ANALYSIS, vol. 1, 01 November 1986, Deerfield Beach, FL (US); G. MICHAL et al., pp. 197-231/
- BIOCHIMICA ET BIOPHYSICA ACTA, vol. 553, 1979, Amsterdam (NL); M. LYNNE et al., pp. 40-53/
- JOURNAL OF BIOLOGICAL CHEMISTRY, vol. 252, no. 10, 25 May 1977, Washington, DC (US); H.J. FORMAN et al., pp. 3379-3381

## Description

This invention relates to a method for the detection of Gram-negative bacteria in liquid samples and to the use of an apparatus using this method.

Microorganisms can be divided into two groups on the basis of the Gram staining test: either Gram-positive or Gram-negative. Their different staining properties are based on differences in their surface structure and the Gram test is therefore an important criterion in the identification of microbes. Particularly in the case of infectious microorganisms, the knowledge of the Gram-reaction is important when choosing an immediate course of action in the case of medication or a form of repellent in the case of environmental hygiene. Gram-staining is traditionally carried out with a sample which has been dried onto an object glass. The dried sample is dyed firstly with crystal violet and then with safranin. As a result Gram-positive bacteria appear blue in microscopical examination and Gram-negative bacteria appear red. The total bacterial count, without the use of a microscope, can also be determined directly from a sample. For this purpose a sample of known volume is collected on the surface of a filter, dyed with safranin and the excess dye is rinsed off. As a result, both Gram-positive and -negative bacteria appear as red spots as described in EP 288621.

The rapid identification of certain groups or species of bacteria from biological samples can also be accomplished using biochemical reactions, as in the case of enzymatic identifications and methods based on carbohydrate utilization. Particularly rapid enzymatic reactions are the oxidation-reduction reactions catalyzed by cytochromes and by enzymes such as the dehydrogenases. These reactions occur in all living cells of both animal and bacterial origin. Dehydrogenases can be assayed from living bacteria without damage to the cells by an established histochemical method, which has been used e.g. for the localization of dehydrogenases in leukocytes and in histological tissue sections (Michel, G. et al., Methods of Enzymatic Analysis, pp. 197-232, vol. 1, 1983). In this method the natural hydrogen acceptor in the dehydrogenase reaction is replaced by e.g. a tetrazolium salt, which becomes blue-violet in colour and also insoluble when reduced by hydrogen. An electron transfer mediator may also be added to the dehydrogenase reaction mixture. Such mediators, e.g. phenazine methosulphate (PMS), serve to accelerate and to amplify the reaction. PMS transfers hydrogen from NAD to the tetrazolium salt, which precipitates as the coloured compound formazan . PMS may also be replaced by other hydrogen acceptors such as menadione, meldola blue (C.I. 51175; Basic blue 6) or methoxy phenazine methosulphate.

The method described above has also been used for the identification of bacteria. When a chemical compound used in the measurement of the oxidation-reduction reaction, such as triphenyltetrazolium chloride (TTC), is added to a sample containing bacteria it is reduced and its colour changes from colourless to red. This change can be measured either visually or by colorimetric measurement. In addition to TTC other, more rapid tetrazolium salts can be used, such as iodonitrotetrazolium (INT) and neotetrazolium chloride (NTC), or alternatively salts which become blue in color, such as blue tetrazolium (BT) and nitroblue tetrazolium (NBT) (Histological and Histochemical Methods, Theory and Practice, Chapter 16, p. 258, second edition 1990).

Different groups of bacteria can be distinguished based on their reaction in the gram-test. Differences between dyeing properties arise as a result of differences in the surface structure of the bacteria. These structural differences also affect the sensitivity of different bacteria to detergents. This property has in turn been utilized in the identification of Gram-negative bacteria in combination with their detection by tetrazolium dyeing as described above.

A suitable concentration of a anionic detergent such as Tergitol-7,selectively inhibits the respiration, i.e. oxygen uptake, of Gram-negative bacteria and consequently also the reduction of tetrazolium to a coloured compound. Cationic detergents inhibit the respiration of both Gram-negative and Gram-positive bacteria, whereas neutral, non-ionic detergents appear to have no effects (Baker et al., J.Exp. Med. 73, 249-271, 1941).

In addition to bacterial respiration, dehydrogenase activity as measured by TTC is also inhibited in the same way (Dakay et al., Zentralblatt Bakt. Hyg. I. Abt. Orig. B: 174 pp. 121-124, 1981). This was demonstrated using four different bacterial strains. These observations have been applied to the identification of bacteria from liquid samples (EP 107594). It was found that anionic detergents which inhibited respiration, prevented also colour formation due to the action of dehydrogenases, whereas certain antibiotics and other growth-inhibiting compounds had no effectsin the same time.

Thus the technical problem underlying the present invention is to provide an improved method for the selective detection of Gram-negative bacteria in liquid samples. The solution to the above technical problem is achieved by providing the embodiments characterized in the claims.

One object of the present invention is a method for the detection of Gram-negative bacteria from a liquid sample such as urine or an industrial process liquid by detecting the presence of dehydrogenase enzymes of Gram-negative bacteria without disrupting the bacteria by using as substrate for the dehydrogenase enzymes redox-compounds changing from colourless to coloured state, in which the Gram-negative bacteria are separated from a suspension using a filter material (3) with a pore size small enough to prevent the passage of Gram-negative bacteria for attainment of maximum sensitivity but large enough to permit the passage of free reducing compounds and the reduction reactions of bacteria other than Gram-negative bacteria are prevented using a combination of a chaotropic and a non-ionic alkyl glucoside-type detergent. The redox-compound is preferably dichloroindophenol, resazurin, triphenyltetrazolium chloride (TTC), iodonitrotetrazolium (INT), neotetrazolium chloride (NTC), blue tetrazolium (BT) or nitroblue tetrazolium (NBT). The chaotropic compound is preferably guanidine or urea. The non-ionic alkyl glucoside-type detergent is preferably octyl glucoside or octyl maltoside.

A further object of the present invention is an apparatus for the detection of Gram-negative bacteria in a liquid sample using the above mentioned method of the present invention, in which the Gram-negative bacteria are separated from a suspension using a filter material (3) with a pore size small enough to prevent the passage of the microorganisms for attainment of optimal sensitivity but large enough to permit the passage of free reducing compounds, and the reduction reactions of bacteria other than Gram-negative bacteria are prevented using either chaotropic or non-ionic detergents or their mixture; and the filter material in the apparatus is situated in immediate contact with an absorbent material so that the absorbent material facilitates the passage of liquid through the filter. The pore size of the filter (3) is preferably 0.75-1.2 µm.

The colour formation caused by the action of dehydrogenases, which serves to indicate the presence of bacteria in a sample, can be directly assayed from the sample without destroying the bacteria. Furthermore, Gram-negative bacteria can be distinguished from the overall bacterial population using a combination of a non-ionic alkyl glucoside-type detergent and a caotropic compound such as guanidine. Other non-ionic detergents such as Triton X-100 and Tween™ 80, which are polyoxyethylene compounds, have no effects under the same conditions, as was reported much earlier by Dakay et al. (1941 supra). The method is performed at room temperature and is particularly suitable when the biological sample is filtered so that the bacteria remain on the filter membrane. The bacteria are dyed immediately on the filter with a light yellow NBT solution, which upon reduction forms a visible blue precipitate on the filter. The blue colour of recuded NBT is formed considerably more rapidly than the red colour of TTC and is easier to distinguish from the red colour of samples containing hemoglobin. NBT and MTT are equally rapid with respect to colour formation, but the advantage of NBT in comparison with MTT is that the colour formation occurs in a restricted area of the filter because the colour precipitates at the place where the dehydrogenases are located. If the bacteria are not disrupted the formazan is located around the bacteria. Excess NBT does not dissolve the blue formazan , whereas MTT is water-soluble and can be used in spectrophotometric methods. Using e.g. NBT bacteria can be identified quantitatively and qualitatively, not only sufficiently rapidly but also without any separate measuring equipment, by simple visual inspection, from biological samples such as urine, milk, water and process solutions when choosing a suitable maintenance or preventive method. In this case yeasts, which are not classified according to Gram type, are dyed in the total microbial determination but not in the estimation of Gram-bacteria.

The method is particularly suited for use in an apparatus in which the bacteria are assayed directly from biological samples. The apparatus comprises a porous filter or a filter composed of several units with different pore sizes, onto which the sample is transferred e.g. by suction. Microorganisms are detected immediately, on the basis of the colour change resulting from oxidation-reduction reactions, on the surface of the filter on which the bacteria were retained. Correspondingly, the amount of Gram-negative bacteria can be determined in a similar way using a suitable combination of detergents. Cells with different particle sizes, such as leucocytes, which are also reduced under the same conditions, can be separted on a different filter or using several filters and assayed individually from each filter. Free colour-reducing compounds possibly present in the sample, such as soluble enzymes, ascorbic acid, pharmaceutical compounds, low molecular weight- sulfhydrylcontaining molecules, e.g. glutathione, and other reducing compounds pass through the filter on which the bacteria are trapped.

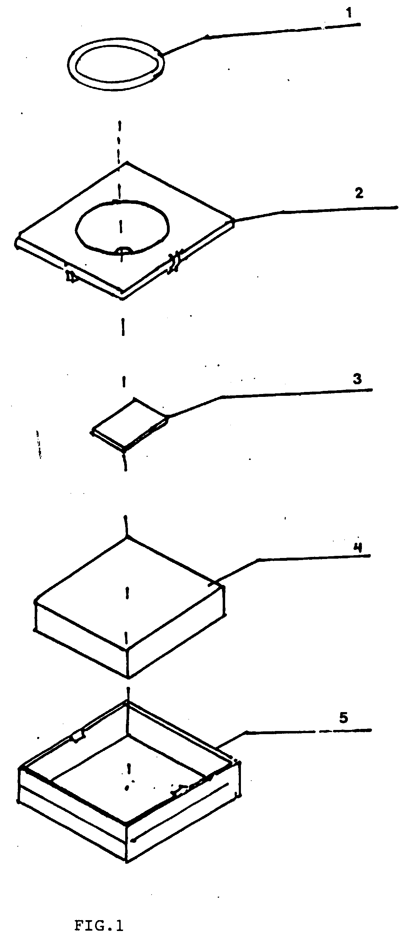

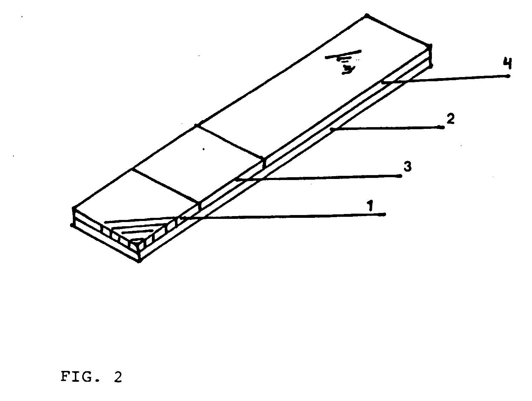

The invention is described in detail in the following with reference to the enclosed figures. Figure 1 represents the apparatus used to collect bacteria from the sample on the filter and Figure 2 the apparatus in which the sample is sucked into the filter. The amount of Gram-negative bacteria can be directly determined with both pieces of the apparatus on the basis of colour changes measuring dehydrogenase activities, whereas the reduction reactions of microbes other than Gram-negative bacterial can be inhibited by the choice of suitable detergent(s).

Figure 1 presents an exploded view of an apparatus in which a liquid sample is pipetted onto the prefilter 1 if the sample contains large particles or if it contains large numbers of e.g. leukocytes (in other cases prefiltration is not necessary) which may prevent visualizing the bacteria. In the collection chamber 2 the microbes in the sample are trapped on the filter 3.

The sample may be pre-buffered with a solution containing (a) detergent(s) to prevent the reduction reactions of all other microbes except Gram-negative bacteria. Alternatively the bacteria trapped on the surface of the filter 3 can be washed with a detergent or with a buffer solution containing detergents and immediately dyed with a mixture for the detection of dehydrogenases. As a result, a blue zone due to the precipitation of formazan is formed around the bacteria. Below the filter 3 is an absorbent material 4, which absorbs the sample liquid. All the parts of the apparatus can be assembled in the base 5, in which the apparatus takes the form of a small box.

The apparatus depicted in Figure 2 includes a prefilter 1 for removing particles from the sample, a filter 3 to which bacteria are moved from the prefilter and on which bacteria are trapped and an absorbent material 4, all assembled an the base 2, which may be made e.g. of plastic. The filters 1 and 3 and the absorbent material 4 may be attached e.g. using two-sided tape to the base 2. The sample, buffered with a solution containing (a) detergent(s), is passed through the prefilter 1 and the bacteria present in the sample are collected in the interface between it and the filter 3, from where they can be detected by passing through the prefilter 1 a mixture for the determination of dehydrogenases . As a result, blue formazan is produced around the Gram-negative bacteria.

### EXAMPLE 1

The test bacteria were isolated from urinary tract infections, either ATCC type strains or clinical strains (E. coli a,b, 2956,3338, and 110515) obtained from the Department of Serology of the Universty of Helsinki. The Gram-negative bacteria were Escherichia coli (ATCC 25922) and several clinical strains: Klebsiella aerogenes (ATCC 13833), Proteus mirabilis, Pseudomonas aeruginosa and Enterobacter aerogenes (ATCC 13048). Gram-positive strains included Staphylococcus aureus (ATCC 25923), Staphylococcus saprophyticus, Staphylococcus epidermidis, Streptococcus agalactiae, Streptococcus faecium (ATCC 9790), B-hemolytic Streptococcus group B serotype, Enterococcus sp. and Corynebacterium sp. The yeast strains were Candida albicans ATCC 28367 and 36802. The bacteria were cultivated overnight in BHI broth (Brain-Heart Infusion, Difco, Detroit) at 37 °C and the bacterial count in the broth was adjusted to about 10⁸ CFU/ml (colony forming units / ml) by dilutionwith a sterile 0.9 % sodium chloride solution to an optical density of 0.400 at 650 nm (Perkin Elmer Spectrophotometer, Norwalk). The bacterial population was checked by dilution and plating on blood agar. The yeast strains were cultivated for 2 days at 37 °C on Sabouraud agar plates and suspensions of 10⁸ CFU/ml were made by transferring the microbial mass to sterile salt solution to an optical density of 0.400 as described above. In addition to these samples, 100 urine samples were collected. The bacterial contents of these samples were assayed by bacterial cultivation (Uricult®, Orion Diagnostica). Leucocytes possibly present in the samples were collected on a leucocyte filter (Pall Biosupport Company, Glen Cove).

In the assay 100 µl ofa bacterial suspension were added to 100 µl 0.2 M Tris-HCl buffer, pH 6.5, 7.2 or 8.5 (Sigma, St. Louis) containing 1.0% glucose (BDH, Poole) or fructose (BDH, Poole), acetate (Merck, Darmstadt) or glutamate (Merck, Darmstadt). For some assays the mixture also contained varying amounts of octyl glucoside (Sigma, St. Louis) or guanidine hydrochloride (BDH, Poole) and was incubated for different times on a 96-well plate. The sample was pipetted to a filtering apparatus, on which the bacteria were collected in an area of about 3 mm diameter on the surface of the filter (Schleicher and Schuell glass fiber filter No. 8), or the filter (leucocyte filter, Pall BioSupport Company, Glen Cove) was dipped into the sample and the bacteria passed through it and transferred to the filter interface where another filter (Schleicher and Schuell No. 8) was positioned. The bacteria were detected by adding 100 µl NBT-solution (1 mg/ml, Sigma, St. Louis) containing 10 µl PMS (1 mg/ml, Sigma, St. Louis) to the filter.

Colour formation was estimated visually after different times according to the scale:
0= colourless; 1= weak colour;
2= clearly detectable colour;
3= strongly coloured; 4= darkly coloured; 5= very darkly coloured.

### EXAMPLE 2

The detection of different clinical strains of Escherichia coli was demonstrated on a filter at different concentrations. The bacteria were incubated for 30 seconds in 0.2 M Tris-HCl buffer, pH 7.2 containing 1.0% glucose and transferred to a filter to which NBT-PMS solution was added and the intensity of the blue colour of formazane was estimated after 30 minutes.

| Clinical bacterial strains Bacteria count / ml (see Example 1) | | | | | |
|---|---|---|---|---|---|
| | 10⁹ | 10⁸ | 10⁷ | 10⁶ | 10⁵ |
| E.coli a | 5 | 5 | 4 | 2 | 0 |
| E.coli b | 5 | 4 | 3 | 2 | 0 |
| E.coli 2956 | 4 | 4 | 3 | 1 | 0 |
| E.coli 3338 | 5 | 5 | 5 | 2 | 0 |
| E.coli 110515 | 4 | 4 | 3 | 0 | 0 |

### EXAMPLE 3

The prevention of formazan production was demonstrated by a Gram-positive bacterium, Staphylococcus, without effecting on the Gram-negative E. coli. The test was performed as described in Example 1 with and without the addition of 0.5 M guanidine.

| | Escherichia coli (ATCC 25922) | | | | | | Staphylococcus saprofyticus | | | | | |
|---|---|---|---|---|---|---|---|---|---|---|---|---|
| | no additives | | | guanidine | | | no additives | | | guanidine | | |
| pH | 6.5 | 7.2 | 8.5 | 6.5 | 7.2 | 8.5 | 6.5 | 7.2 | 8.5 | 6.5 | 7.2 | 8.5 |
| bact./ml | | | | | | | | | | | | |
| 10 ⁷ | 2 | 3 | 3 | 2 | 3 | 3 | 4 | 4 | 4 | 0 | 0 | 1 |
| 10 ⁶ | 2 | 2 | 2 | 2 | 2 | 2 | 0 | 2 | 3 | 0 | 0 | 0 |
| 10 ⁵ | 0 | 0 | 0 | 0 | 0 | 0 | 0 | 0 | 0 | 0 | 0 | 0 |

### EXAMPLE 4

The prevention of formazan production was demonstrated by different bacteria with 0.5 M guanidine at pH 7.2, in the presence of different substrates: 1 % glucose; 1 % fructose; 1 % glutamate; 1 % acetate. In other respects the test was carried out as described in Example 1.

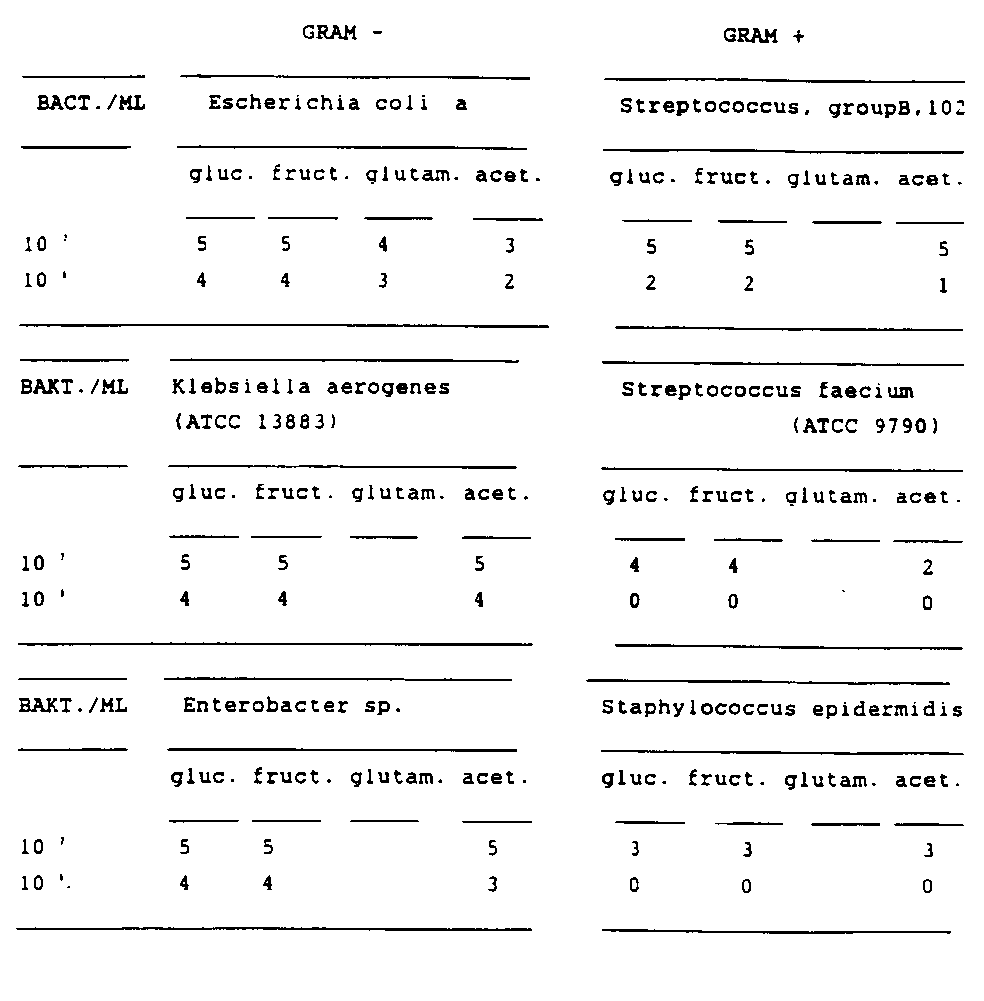

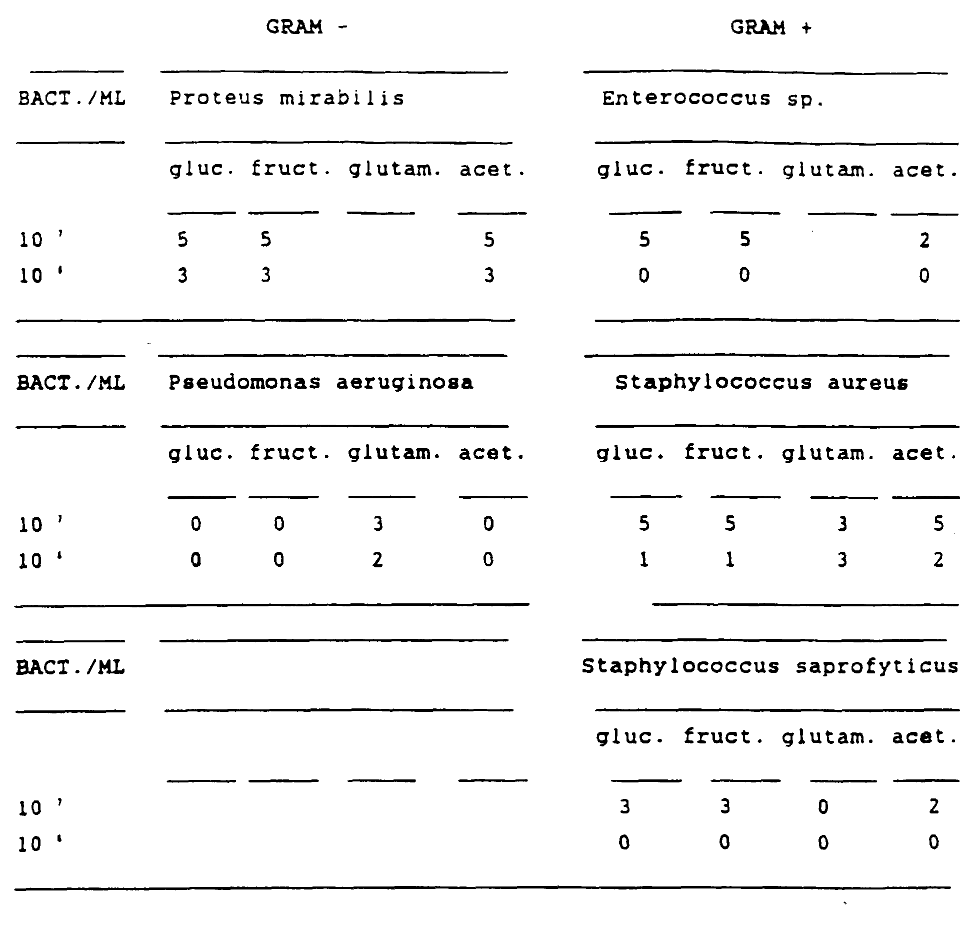

### EXAMPLE 5

The prevention of formazan production was demonstrated by different bacteria with octyl glucoside at different concentrations. In other respects the test was carried out as described in Example 1, except that the bacterial concentration was 10⁷ CFU/ml.

| | octylglucoside concentration (g/l) | | | |
|---|---|---|---|---|
| GRAM - | 4 | 6 | 8 | 10 |
| Escherichia coli a | 4 | 5 | 4 | 4 |
| Klebsiella aerogenes | 5 | 5 | 5 | 5 |
| Enterobacter aerogenes | 5 | 5 | 5 | 3 |
| Proteus mirabilis | 5 | 2 | 2 | 2 |
| Pseudomonas aeruginosa | 0 | 0 | 0 | 0 |

| GRAM + | | | | |
|---|---|---|---|---|
| Staphylococcus aureus | 5 | 0 | 0 | 0 |
| Enterococcus sp. | 4 | 0 | 0 | 0 |
| Staphylococcus epidermidis | 3 | 3 | 0 | 0 |
| Staphylococcus saprophyticus | 3 | 2 | 2 | 2 |
| Streptococcus agalactiae | 4 | 0 | 0 | 0 |
| Streptococcus faecium | 3 | 0 | 0 | 0 |
| Streptococcus sp. B | 5 | 0 | 0 | 0 |

### EXAMPLE 6

The prevention of formazan production was demonstrated by different bacteria using 0.5 M guanidine with or without 10 g/l octyl glucoside. In other respects the test was performed as described in Example 1.

| GRAM - | octylglucoside added | octylglucoside not added |
|---|---|---|
| Escherichia coli a | 4 | 5 |
| Klebsiella aerogenes | 4 | 5 |
| Enterobacter aerogenes | 4 | 5 |
| Proteus mirabilis | 5 | 5 |
| Pseudomonas aeruginosa | 4 | 0 |

| GRAM + | | |
|---|---|---|
| Staphylococcus aureus | 0 | 5 |
| Enterococcus sp. | 0 | 5 |
| Staphylococcus epidermidis | 0 | 4 |
| Staphylococcus saprofyticus | 0 | 4 |
| Streptococcus agalactiae | 0 | 5 |
| Streptococcus faecium | 0 | 4 |
| Streptococcus sp. B | 1 | 5 |
| Corynebacterium sp. | 0 | 5 |
| Candida albicans | 0 | 4 |
| Candida albicans B | 0 | 3 |

### EXAMPLE 7

The cultivation of 100 urine samples was carried out with Uricult® and the results were compared with the method described in Example 2, in which bacteria were detected with formation of formazan. A positive result (+) of the Uricult® test indicates that the sample contains at least 10⁵ bacteria/ml. Bacterial counts below 10⁵ CFU/ml give a negative result (-). A positive result with formazan was obtained when the colour intensity was 2 or greater and a negative result was indicated by a colour intensity of 0 or 1.

| | | formation of formazan | |
|---|---|---|---|
| | | + | - |
| Total bacterial count, Uricult | + | 21 | 0 |
| | - | 0 | 79 |
| GRAM - reaction, Uricult | + | 12 | 1 |
| | - | 0 | 87 |
| GRAM + reaction, Uricult | + | 6 | 2 |
| | - | 0 | 92 |

## Claims

1. A method for the detection of Gram-negative bacteria comprising
(A) the separation of said Gram-negative bacteria without disruption from a liquid sample using a filter material (3), and
(B) a colour-forming reaction caused by said Gram-negative bacteria on the filter material (3), wherein said reaction comprises
(a) at least one enzyme derived from said Gram-negative bacteria on the filter material (3),
(b) at least one compound capable of changing its colour upon reaction with said enzyme,
(c) at least one chaotropic compound, and
(d) at least one non-ionic alkyl glucoside-type detergent.

2. The method according to claim 1, wherein the non-ionic alkyl glucoside-type detergent is octyl glucoside or octyl maltoside.

3. The method according to any one of claims 1 to 2, wherein the reaction further comprises at least one electron transfer mediator.

4. The method according to claim 3, wherein the mediator is phenazine methosulphate, menadione, meldola blue, or methoxy phenazine methosulfate.

5. The method according to any one of claims 1 to 4, wherein the liquid sample is urine or an industrial process liquid.

6. The method according to any one of claims 1 to 5, wherein the enzyme is a dehydrogenase.

7. The method according to any one of claims 1 to 6, wherein the compound is a redox-compound.

8. The method according to claim 7, wherein the redox-compound is dichloroindophenol, resazurin, triphenyltetrazolium chloride (TTC), iodonitro-tetrazolium (INT), neotetrazolium chloride (NTC), blue tetrazolium (BT) or nitroblue tetrazolium (NBT).

9. The method according to any one of claims 1 to 8, wherein the chaotropic compound is guanidine or urea or a salt thereof.

10. Use of an apparatus comprising
(a) a filter material (3), and
(b) an absorbent material (4),
wherein the filter material (3) is placed in immediate contact with the absorbent material (4) for facilitating the passage of a liquid sample through the filter material (3), for the detection of Gram-negative bacteria according to the method of any one of claims 1 to 9.

11. Use according to claim 10, whereby the apparatus further comprises (c) a prefilter (1).

12. Use according to claim 10 or 11, whereby the filter material (3) comprises at least one filtration unit having a pore size ranging from 0.75 µm to 1.2 µm.

## Patentansprüche

1. Verfahren zum Nachweis gramnegativer Bakterien, umfassend:
(A) die Abtrennung der gramnegativen Bakterien ohne Aufschluß von einer flüssigen Probe unter Verwendung eines Filtermaterials (3), und
(B) eine farbgebende Reaktion, die durch die gram-negativen Bakterien auf dem Filtermaterial (3) hervorgerufen wird und umfaßt
(a) mindestens ein Enzym, das von den gramnegativen Bakterien auf dem Filtermaterial (3) stammt,
(b) mindestens eine Verbindung, die nach der Reaktion mit dem Enzym die Farbe ändern kann,
(c) mindestens eine chaotrope Verbindung, und
(d) mindestens ein nicht-ionisches Detergens vom Alkylglukosid-Typ.

2. Verfahren nach Anspruch 1, wobei das nicht-ionische Detergens vom Alkylglukosid-Typ ein Octylglukosid oder ein Octylmaltosid ist.

3. Verfahren nach Anspruch 1 oder 2, wobei die Reaktion außerdem mindestens einen Elektronentransfer-Mediator umfaßt.

4. Verfahren nach Anspruch 3, wobei der Mediator Phenazinmethosulfat, Menadion, Meldola-Blau oder Methoxyphenazinmethosulfat ist.

5. Verfahren nach einem der Ansprüche 1 bis 4, wobei die flüssige Probe Urin oder eine von einem industriellen Prozess stammende Flüssigkeit ist.

6. Verfahren nach einem der Ansprüche 1 bis 5, wobei das Enzym eine Dehydrogenase ist.

7. Verfahren nach einem der Ansprüche 1 bis 6, wobei die Verbindung eine Redoxverbindung ist.

8. Verfahren nach Anspruch 7, wobei die Redoxverbindung Dichlorindophenol, Resazurin, Triphenyltetrazoiium-chlorid (TTC), Iodonitrotetrazolium (INT), Neo-tetrazoliumchlorid (NTC), Blautetrazolium (BT), oder Nitroblautetrazolium (NBT) ist.

9. Verfahren nach einem der Ansprüche 1 bis 8, wobei die chaotrophe Verbindung Guanidin, Harnstoff oder ein Salz davon ist.

10. Verwendung einer Apparatur, umfassend
(a) ein Filtermaterial (3), und
(b) ein absorbierendes Material (4),
wobei das Filtermaterial (3) in direkten Kontakt mit dem absorbierenden Material (4) gebracht wird, um den Durchlauf einer flüssigen Probe durch das Filtermaterial (3) zu erleichtern, für den Nachweis von gramnegativen Bakterien gemäß dem Verfahren nach einem der Ansprüche 1 bis 9.

11. Verwendung nach Anspruch 10, wobei die Apparatur außerdem (c) einen Vorfilter (1) umfaßt.

12. Verwendung nach Anspruch 10 oder 11, wobei das Filtermaterial (3) mindestens eine Filtrationseinheit mit einer Porengröße zwischen 0.75 µm und 1.2 µm umfaßt.

## Revendications

1. Procédé pour la détection de bactéries Gram négatives comprenant
(A) la séparation de bactéries Gram négatives sans lyse, à partir d'un échantillon liquide, au moyen d'un matériau filtrant (3), et
(B) une réaction de coloration provoquée par lesdites bactéries Gram négatives sur le matériau filtrant (3), ladite réaction comprenant
(a) au moins une enzyme provenant desdites bactéries Gram négatives présentes sur le matériau filtrant (3),
(b) au moins un composé capable de changer de couleur lors de la réaction avec ladite enzyme,
(c) au moins un composé chaotrope, et
(d) au moins un détergent non ionique de type alkylglucoside.

2. Procédé selon la revendication 1, dans lequel le détergent non ionique de type alkylglucoside est l'octylglucoside ou l'octylmaltoside.

3. Procédé selon la revendication 1 ou 2, dans lequel la réaction comprend en outre au moins un médiateur de transfert d'électrons.

4. Procédé selon la revendication 3, dans lequel le médiateur est le méthosulfate de phénazine, la ménadione, le bleu Meldola ou le méthosulfate de méthoxyphénazine.

5. Procédé selon l'une quelconque des revendications 1 à 4, dans lequel l'échantillon liquide est l'urine ou un liquide de processus industriel.

6. Procédé selon l'une quelconque des revendications 1 à 5, dans lequel l'enzyme est une déshydrogénase.

7. Procédé selon l'une quelconque des revendications 1 à 6, dans lequel le composé est un composé rédox.

8. Procédé selon la revendication 7, dans lequel le composé rédox est le dichloro-indophénol, la résazurine, le chlorure de triphényltétrazolium (CTT), l'iodonitrotétrazolium (INT), le chlorure de néotétrazolium (NTC), le bleu de tétrazolium (BT) ou le bleu nitré de tétrazolium (BNT).

9. Procédé selon l'une quelconque des revendications 1 à 8, dans lequel le composé chaotrope est la guanidine ou l'urée, ou un sel de celles-ci.

10. Utilisation d'un appareil comprenant
(a) un matériau filtrant (3) et
(b) un matériau absorbant (4), le matériau filtrant (3) étant mis en contact immédiat avec le matériau absorbant (4) pour faciliter le passage d'un échantillon liquide à travers le matériau filtrant (3),
pour la détection de bactéries Gram négatives selon le procédé de l'une quelconque des revendications 1 à 9.

11. Utilisation selon la revendication 10, dans laquelle l'appareil comprend en outre (c) un préfiltre (1).

12. Utilisation selon la revendication 10 ou 11, dans laquelle le matériau filtrant (3) comprend au moins une unité de filtration ayant une taille de pores allant de 0,75 µm à 1,2 µm.
